# EUROPEAN PATENT APPLICATION

(11) **EP 4 813 359 A1**
(43) Date of publication of application: **30.09.2026**
(21) Application number: 23959062.3
(22) Date of filing: 22.11.2023
(51) Int. Cl.: A61F 7/03

(54) **HEATING IMPLEMENT**

(71) Applicant: Kao Corporation, Tokyo 103-8210 (JP)
(72) Inventor: WATANABE, Yuki, Tokyo 103-8210 (JP); ENDO, Youichi, Tokyo 103-8210 (JP); MINAGAWA, So, Tokyo 103-8210 (JP)
(74) Representative: Hoffmann Eitle
(86) International application number: PCT/JP2023/042074
(87) International publication number: WO 2025/109734

(57) **Abstract**

Provided is a warming device (10), in which a carbon component and water are contained, a water-retention layer (2) includes one, or two or more selected from: a dispersion layer that is a layer of a thermoplastic resin in which a water-absorbent polymer is dispersed; and a layer of a thermoplastic water-absorbent resin, the water-retention layer (2) retains water, a heat-generating layer (1) and the water-retention layer (2) are adjacent to each other and are disposed in a closed space sandwiched between a first sheet (3) and a second sheet (4), the first sheet (3) is air-permeable, and in a plan view, the heat-generating layer (1) and the water-retention layer (2) are smaller in area than the first sheet (3) and the second sheet (4), and a flap portion where the first sheet (3) and the second sheet (4) overlap is provided outside outer peripheral edges of the heat-generating layer (1) and the water-retention layer (2).

## Description

### Technical Field

The present invention relates to a warming device.

### Background Art

Various proposals have been made on warming devices utilizing oxidation heat generated by an oxidation reaction of an oxidizable metal.

For example, Patent Literature 1 describes a sweat-absorbent warming-body structure in which a heat generating element composition is enclosed in a flat bag body. The support on the back surface of the flat bag body is made of a laminated film in which a water-absorbing layer is interposed between a synthetic resin film and a porous synthetic resin film, and a pressure-sensitive adhesive layer is partially formed on the exposed surface of the porous synthetic resin film. This support ensures adhesion to the skin at the site of the pressure-sensitive adhesive layer and allows sweat and waste products to be absorbed into the water-absorbing layer at the site where the pressure-sensitive adhesive layer is not present and can clean the skin surface.

In addition, Patent Literature 2 describes a warming device in which a steam generator obtained by laminating a heat-generating layer and a water-absorbent sheet is accommodated in an air-permeable bag body. The heat-generating layer contains a water-absorbent polymer, in which the mass ratio of the water-absorbent sheet to the water-absorbent polymer is 0.9 or more and 15 or less. This allows good rise of temperature and can appropriately improve the amount of water vapor generated. The literature describes that the water-absorbent sheet in the warming device is paper or nonwoven fabric made of a fiber material, or a laminate of paper and nonwoven fabric.

### Citation List

### Patent Literature

Patent Literature 1: JP H03-100090 A
Patent Literature 2: JP 2017-023712 A

The description of Background Art presented herein is for the purpose of generally presenting the context of the disclosure. To the extent that work of the presently named inventors is described in this Background Art section, such work, as well as aspects of the description that may not otherwise qualify as prior art at the time of filing, are not admitted, either expressly or impliedly, as prior art against the present invention.

### Summary of Invention

The present invention provides a warming device including a first sheet, a second sheet, a heat-generating layer, and a water-retention layer.

Preferably, the heat-generating layer contains an oxidizable metal, a carbon component, and water.

Preferably, the water-retention layer includes one, or two or more selected from: a dispersion layer that is a layer of a thermoplastic resin in which a water-absorbent polymer is dispersed; and a layer of a thermoplastic water-absorbent resin.

Preferably, the water-retention layer retains water.

Preferably, the heat-generating layer and the water-retention layer are adjacent to each other.

Preferably, the heat-generating layer and the water-retention layer are disposed in a closed space that is sandwiched between the first sheet and the second sheet.

Preferably, the first sheet is air-permeable.

Preferably, in a plan view of the warming device, the heat-generating layer and the water-retention layer are smaller in area than the first sheet and the second sheet.

Preferably, a flap portion where the first sheet and the second sheet overlap is provided outside outer peripheral edges of the heat-generating layer and the water-retention layer.

In addition, the present invention provides a method for manufacturing a warming device.

Preferably, the method includes applying a coating material containing an oxidizable metal, a carbon component, and water onto one surface of the first sheet.

Preferably, the method includes applying: a slurry of a molten thermoplastic resin containing water-absorbent polymer particles; and/or a molten thermoplastic water-absorbent resin onto one surface of the second sheet.

Preferably, the manufacturing method includes bonding the two sheets in such a way that their coated surfaces face each other.

Preferably, in the manufacturing method, one or both of the first sheet and the second sheet are air-permeable sheets.

The above-described and other features and advantages of the present invention will become more apparent from the following description with reference to the accompanying drawings as appropriate.

### Brief Description of Drawings

[Fig. 1] Fig. 1 is a cross-sectional view schematically illustrating a preferred embodiment of the warming device of the present invention.
[Fig. 2] Fig. 2(A) is a cross-sectional view schematically illustrating another preferred embodiment of the warming device of the present invention. Fig. 2(B) is a plan view illustrating an example in which a water-retention layer in the embodiment of Fig. 2(A) is arranged in a divided manner.
[Fig. 3] Fig. 3(A) is a cross-sectional view schematically illustrating an example of a water-retention layer including a dispersion layer. Fig. 3(B) is a cross-sectional view schematically illustrating an example of a water-retention layer including a layer of a thermoplastic water-absorbent resin.
[Fig. 4] Fig. 4(A) is a plan view illustrating an example of five locations to be observed in measuring a content mass proportion of a fiber material in the water-retention layer. Fig. 4(B) is a plan view illustrating another example of Fig. 4(A).
[Fig. 5] Fig. 5(A) is a plan view illustrating an example of a location where a cross section of the warming device is prepared to measure a thickness of the water-retention layer. Fig. 5(B) is a cross-sectional view illustrating an example of five locations to be observed in a cross section taken along line 5B-5B illustrated in Fig. 5(A).
[Fig. 6] Fig. 6 is a photograph substituted for a drawing, presenting an example of an embodiment including an arrangement configuration in which adjacent water-absorbent polymers are in contact with each other in a dispersion layer included in a water-retention layer.
[Fig. 7] Fig. 7 is a cross-sectional view illustrating an example of section positions of five locations to be observed in a cross-section of a water-retention layer in measuring a standard deviation of area values of a water-absorbent polymer in the water-retention layer.
[Fig. 8] Fig. 8 is a cross-sectional view schematically illustrating, in an aspect in which the water-retention layer is a dispersion layer and includes a layer having a water-soluble resin, a transition from (A) a state before the water-retention layer absorbs water to (B) a state in which the water-retention layer has absorbed water and formation of a network of a thermoplastic resin and swelling of a water-absorbent polymer have occurred.
[Fig. 9] Fig. 9(A) and Fig. 9(B) are photographs substituted for drawings, presenting an example of a surface state of the dispersion layer on a heat-generating layer side in the state illustrated in Fig. 8(B).
[Fig. 10] Fig. 10(A) and Fig. 10(B) are photographs substituted for drawings, presenting, in an aspect in which the water-retention layer includes a dispersion layer, an example of a state in which the dispersion layer includes cracked portions on a surface on the heat-generating layer side.
[Fig. 11] Fig. 11 is an explanatory diagram illustrating a preferred embodiment of a method for manufacturing a warming device of the present invention.
[Fig. 12] Fig. 12 is an explanatory diagram illustrating another preferred embodiment of a method for manufacturing a warming device of the present invention.
[Fig. 13] Fig. 13 is an explanatory diagram illustrating yet another preferred embodiment of a method for manufacturing a warming device of the present invention.
[Fig. 14] Fig. 14(A) is a cross-sectional view schematically illustrating a warming device sample of Comparative Example 1. Fig. 14(B) is a cross-sectional view schematically illustrating a warming device sample of Comparative Examples 2 and 3.

### Detailed Description of Invention

The present invention relates to providing a warming device capable of achieving a reduced thickness and, at the same time, an increased maximum temperature during heat generation. "During heat generation" as used herein means the entire period during which the warming device generates heat.

The warming device includes: a heat-generating layer containing an oxidizable metal, a carbon component, and water; and a water-retention layer to control the amount of water retained in the heat-generating layer. Specific examples of the control of the amount of water retained by the water-retention layer include absorbing excess water in the heat-generating layer and further supplying water during heat generation of the heat-generating layer. The water-retention layer having such a function is indispensable from the viewpoint of favorably exhibiting the heat generation characteristics of the warming device.

The warming device is used in contact with the skin of a human body and the like and is required to have fit to the skin from the viewpoint of appropriate warmth transfer. From the viewpoint of the fit, the thickness of the warming device is preferably as small as possible. In addition, the thinner warming device is more flexible and, in combination with the fit described above, enhances a good feeling of use on the skin surface in contact with the device. However, reducing the thickness of the water-retention layer to achieve that effect affects the heat generation characteristics of the heat-generating layer in the art and thus has been limited. That is, reducing the thickness of the water-retention layer and achieving good heat generation characteristics have been in a trade-off relationship in the art, making it difficult to achieve both.

In contrast, the warming device of the present invention can achieve a reduced thickness and, at the same time, an increased maximum temperature during heat generation. In addition, according to a method for manufacturing a warming device of the present invention, the warming device of the present invention can be favorably manufactured.

These and other objects of the present disclosure have been achieved by the following discoveries individually or in combination.

Any numerical limit or range described herein includes the end values illustrated in that numerical limit or range. In addition, all values and subranges within the numerical limit or range are expressly included as if explicitly written out.

Singular words and the like used herein have a meaning of "one or a plurality".

Obviously, various modifications and variations of the present invention are possible in view of the disclosure described above and below. Thus, it should be understood that the present invention can be implemented in embodiments not explicitly described herein within the technical scope based on the description of the claims.

The entire contents of the patent literatures and other documents described above and below are fully incorporated herein by reference as if described in detail.

Hereinafter, the present invention will be described based on its preferred embodiments with reference to drawings.

A warming device 10 preferably has a first sheet 3, a second sheet 4, a heat-generating layer 1, and a water-retention layer 2.

The first sheet 3 is typically disposed on the skin side, and the second sheet 4 is typically disposed on the non-skin side. However, the present invention is not limited to this arrangement; for example, the second sheet 4 may be disposed on the skin side.

The heat-generating layer 1 preferably contains an oxidizable metal 11, a carbon component 12, and water.

The water-retention layer 2 preferably includes one, or two or more selected from: a dispersion layer 22S that is a layer of a thermoplastic resin 22 in which a water-absorbent polymer 21 is dispersed; and a layer 23S of a thermoplastic water-absorbent resin 23.

The water-retention layer 2 preferably retains water.

The heat-generating layer 1 and the water-retention layer 2 are preferably adjacent to each other.

The heat-generating layer 1 and the water-retention layer 2 are preferably disposed in a closed space 5 sandwiched between the first sheet 3 and the second sheet 4.

The "closed space 5" as used herein refers to a space whose periphery is sealed so that the constituent materials of the heat-generating layer 1 and the water-retention layer 2 do not leak to the outside of the warming device 10, and can also be referred to as a bag-like space with the opening sealed. The closed space 5 preferably allows a gas, such as air or steam, to enter and exit.

The first sheet 3 is preferably air-permeable.

In a plan view of the warming device 10, the heat-generating layer 1 and the water-retention layer 2 are smaller in area than the first sheet 3 and the second sheet 4.

In a plan view of the warming device 10, a flap portion 6 where the first sheet 3 and the second sheet 4 overlap is preferably provided outside outer peripheral edges of the heat-generating layer 1 and the water-retention layer 2.

More specifically, in a plan view of the warming device 10, the flap portion 6 is preferably a portion where both the first sheet 3 and the second sheet 4 extend outward from the region of the heat-generating layer 1 and the water-retention layer 2 and are overlapped and joined in the thickness direction.

The heat-generating layer 1 and the water-retention layer 2 are preferably not interposed in the flap portion 6.

In a plan view of the warming device 10, a region inside the flap portion 6 is preferably the closed space 5 sandwiched between the first sheet 3 and the second sheet 4.

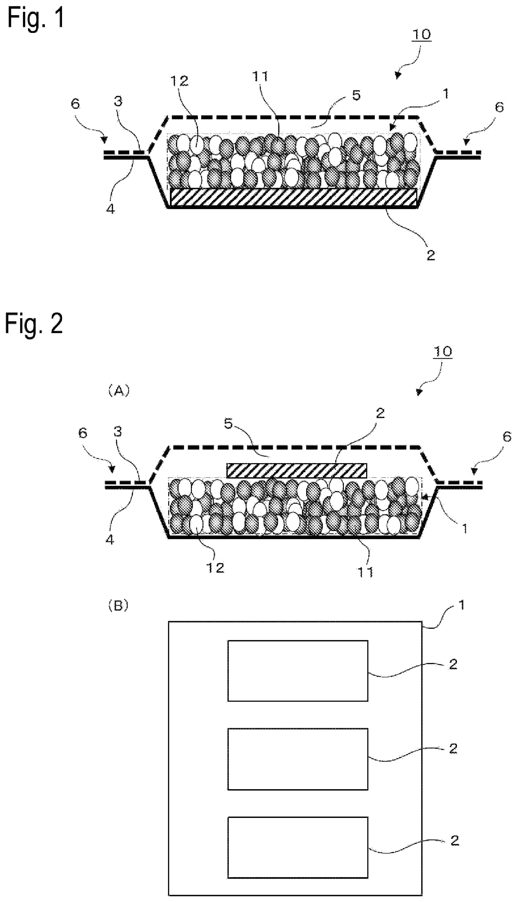

Examples of such embodiments are illustrated in Figs. 1, 2, and 3(A) and 3(B).

When brought into contact with air, the heat-generating layer 1 generates heat by an oxidation reaction of the oxidizable metal 11. At that time, the contained water promotes the oxidation reaction of the oxidizable metal. The carbon component 12 has at least one function of water retention capacity, oxygen supply capacity, or catalytic ability, and promotes the heat generation.

The warming device 10 can apply heat to the skin by the heat generation of the heat-generating layer 1. In addition, in the warming device 10, the water in the heat-generating layer 1 may be heated by the heat generation so as to apply warm vapor to the skin.

As described above, the water-retention layer 2 absorbs the excess water contained in the heat-generating layer 1 and further supplies water to the heat-generating layer 1 during heat generation to control the amount of water retained so as to achieve suitable heat generation characteristics of the heat-generating layer 1.

In the dispersion layer 22S described above, the amount of water retained in the heat-generating layer 1 is controlled by the water absorption capacity of the water-absorbent polymer 21.

In addition, in the layer 23S of the thermoplastic water-absorbent resin 23 described above, the thermoplastic water-absorbent resin 23 itself has a water absorption capacity and controls the amount of water retained in the heat-generating layer 1.

In the case where the water-retention layer 2 includes the dispersion layer 22S described above, the dispersion layer 22S is a continuous layer in which the thermoplastic resin 22 fills the inside of the layer while including the water-absorbent polymer 21.

Similarly, in the case where the water-retention layer 2 includes the layer 23S of the thermoplastic water-absorbent resin 23 described above, the layer 23S is a continuous layer in which the thermoplastic water-absorbent resin 23 fills the inside of the layer.

The water-retention layer 2 including such a layer is preferably a sheet.

The "continuous layer" as used herein is different from a layer in which a plurality of fibers separated from each other is accumulated, such as an assembly of fibers, and refers to a layer in a state filled with the thermoplastic resin 22 or the thermoplastic water-absorbent resin 23 as one mass. The "continuous layer" includes not only a completely continuous state but also a state filled with the resin component to form the entire layer even though the layer includes a crack. In addition, in the dispersion layer 22S, a layer in a state filled with the thermoplastic resin 22 so as to fix the arrangement of the water-absorbent polymer 21 is referred to as the "continuous layer". The water-absorbent polymer 21 is a surface-crosslinked polymer material, what is called a superabsorbent polymer (SAP).

The water-retention layer 2 includes the continuous layer of the thermoplastic resin 22 or the thermoplastic water-absorbent resin 23 as described above, thus reducing gaps between fibers such as those in fiber assemblies known in the art. Thus, the water-retention layer 2 significantly reduces its thickness compared with a fiber layer, enabling thickness reduction.

At the same time, the configuration of the water-retention layer 2 enables uniform water absorption capacity. The "uniform water absorption capacity" as used herein means the water absorption capacity mainly in a planar direction.

In the dispersion layer 22S, the dispersed arrangement of the water-absorbent polymer 21 is typically fixed. This can prevent uneven distribution of the water-absorbent polymer due to movement after manufacturing a heating device, thereby reducing the fluctuation of the water absorption capacity of the water-retention layer 2 and enabling uniform water absorption capacity. In addition, in the example of the manufacturing method described later, the water-retention layer 2 is preferably formed by dispersing the water-absorbent polymer 21 in a slurry of the thermoplastic resin 22 and applying the resulting dispersion. Forming the water-retention layer 2 by this treatment makes it easier to control the dispersed arrangement of the water-absorbent polymer and to achieve uniform water absorption capacity in the resulting water-retention layer 2.

Furthermore, in the layer 23S of the thermoplastic water-absorbent resin 23, the thermoplastic water-absorbent resin 23 itself absorbs water, and thus the entire layer exhibits the water absorption capacity. This reduces the fluctuation of the water absorption capacity of the water-retention layer 2, enabling uniform water absorption capacity as described above.

The water-retention layer 2 thus exhibits uniform water absorption capacity and can control the amount of water retained in the entire heat-generating layer 1 adjacent to the water-retention layer 2. As a result, the warming device 10 includes the water-retention layer 2 with reduced thickness and has reduced overall thickness, and at the same time, can achieve stable and good heat generation characteristics. The uniform control of the amount of water retained in the heat-generating layer 1 by the water-retention layer 2 enables the heat generation, for example, at a maximum temperature of 45°C or higher in a measurement in accordance with JIS S4100.

As described above, the warming device 10 of the present embodiment can achieve a reduced thickness and, at the same time, an increased maximum temperature during heat generation.

The dispersion layer 22S and the layer 23S of the thermoplastic water-absorbent resin 23 in the water-retention layer 2 preferably have elasticity. This provides the warming device 10 with improved flexibility and fit to the skin due to the reduced thickness as well as a reassuring texture with a certain elasticity even with the reduced thickness.

Furthermore, the "thermoplasticities" of the thermoplastic resin 22 and the thermoplastic water-absorbent resin 23 in the water-retention layer 2 can be softened with the heat generation of the heat-generating layer 1. This enhances the softness of the warming device 10 during use more than before use and increases the soft texture described above as time elapses during use.

In the warming device 10 of the present embodiment, the heat-generating layer 1 and the water-retention layer 2 are preferably laminated in the thickness direction of the warming device 10. The order of the lamination can be determined as appropriate.

For example, the air-permeable first sheet 3 and the heat-generating layer 1 may be disposed so as to be in contact with each other. In this case, the water-retention layer 2 adjacent to the heat-generating layer 1 is typically disposed in contact with the second sheet 4. In the case of using the warming device 10 with the first sheet 3 facing the skin side, the heat generated by the heat-generating layer 1 can be more effectively applied to the skin via the first sheet 3 without being blocked by the water-retention layer 2. In addition, the short distance between the heat-generating layer 1 and the skin makes it easier to transfer the heat to the skin. An example of this embodiment is illustrated in Fig. 1.

Furthermore, the water-retention layer 2 may be disposed so as to be in contact with the first sheet 3. In this case, in a plan view from the first sheet 3 side, an area of the water-retention layer 2 is preferably smaller than an area of the heat-generating layer 1. Using the warming device 10 with the first sheet 3 facing the skin side reduces the air permeation inhibition by the water-retention layer 2 and more favorably enables the heat-generating layer 1 to generate heat and apply the heat to the skin. An example of this embodiment is illustrated in Figs. 2(A) and 2(B). As illustrated in Fig. 2(A), the width of the water-retention layer 2 may be smaller than the width of the heat-generating layer 1. Alternatively or additionally, as illustrated in Fig. 2(B), the water-retention layer 2 may be formed into a plurality of belt-like bodies and intermittently arranged on the plane of the heat-generating layer 1.

In the case where the water-retention layer 2 is in contact with the first sheet 3, from the viewpoint of more effectively avoiding air permeation inhibition by the water-retention layer 2, a ratio (M2/M1) of an area M2 of the water-retention layer 2 to an area M1 of the heat-generating layer 1 is preferably 1.2 or less, more preferably 1.1 or less, and even more preferably 1.0 or less.

From the viewpoint of more effectively controlling the amount of water retained in the heat-generating layer 1 by the water-retention layer 2, the ratio (M2/M1) is preferably 0.2 or more, more preferably 0.3 or more, and even more preferably 0.5 or more.

In the warming device 10 of the present embodiment, the water-retention layer 2 preferably has a small content of a fiber material from the viewpoint of more suitably achieving the thickness reduction described above.

From this viewpoint, a plan view area of the fiber material in the water-retention layer 2 is 0% or more and 10% or less, preferably 5% or less, and more preferably 3% or less relative to a plan view area of an entire water-retention layer 2. In addition, the proportion of the plan view area of the fiber material is preferably 0%.

### Method for Measuring Proportion of Plan View Area of Fiber Material relative to Plan View Area of Entire Water-Retention Layer 2

### (i) Pretreatment

First, after 5 minutes have elapsed from the start of the exothermic reaction of the warming device 10 to be measured, such as by opening the packaging bag, the first sheet 3 and the second sheet 4 extending outward in a planar direction of the heat-generating layer 1 and the water-retention layer 2 are cut so as not to cut the heat-generating layer 1 and the water-retention layer 2. Next, the sheet (the first sheet 3 in the laminated form illustrated in Fig. 1 or the second sheet 4 in the laminated form illustrated in Fig. 2) covering the outer surface of the heat-generating layer 1 is removed, and the heat-generating layer 1 is further removed with a spatula. At this time, from the opening of the packaging bag to the removal of the heat-generating layer 1 with a spatula, the work is performed under a nitrogen atmosphere with an oxygen concentration of 3% or less.

### (ii) Measurement

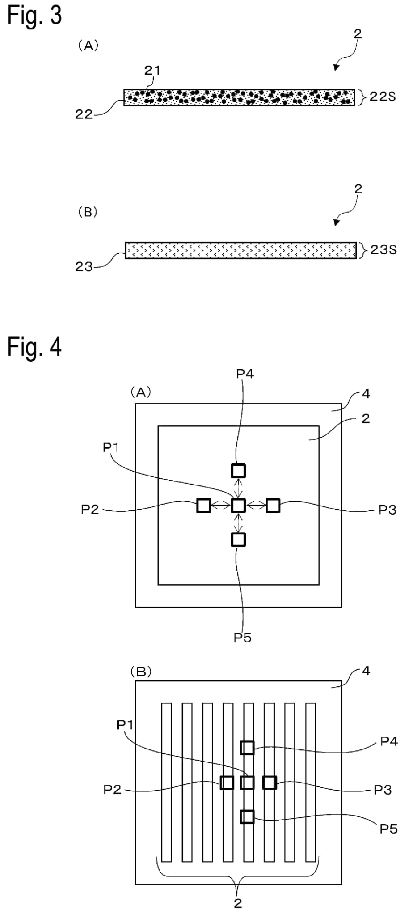

Next, the surface of the water-retention layer 2 is observed and imaged using a microscope (VHX-5000, available from Keyence Corporation). At this time, a 1-mm² square of the center portion of the water-retention layer 2 and 1-mm² squares each at a location spaced apart by 10 mm from each of the four sides of the 1-mm² square of the center portion are cut out, and a total of five locations are observed and imaged. For example, in the water-retention layer 2 having a continuous plane as illustrated in Fig. 4(A), center P1 of the surface area and locations P2 to P5 surrounding center portion P1 are observed and imaged. In addition, for example, in the case where the water-retention layer 2 is an assembly of a plurality of stripe shapes as illustrated in Fig. 4(B), a 1-mm² square of central portion P1 of the central stripe of the assembly, 1-mm² squares of corresponding locations P2 and P3 of the stripes adjacent to the left and right of the central stripe having P1, and 1-mm² squares of locations P4 and P5 spaced apart above and below from P1 by 10 mm are observed and imaged. Figs. 4(A) and 4(B) illustrate the case of the laminated form illustrated in Fig. 1, but in the case of the laminated form illustrated in Fig. 2, the sheet under the water-retention layer 2 is the first sheet 3 in the above measurement.

Next, area ratios of the fiber material to others are calculated using image editing software. The area is the average value of the five locations. The area is measured over the entire 1-mm² square.

In this measurement, ImageJ (open source) is used as the image editing software.

From the viewpoint of further enhancing the good feeling of use of the warming device 10, the thickness of the water-retention layer 2 is preferably 500 µm or less, more preferably 400 µm or less, and even more preferably 300 µm or less. The water-retention layer 2 having a continuous layer of the thermoplastic resin 22 and/or the thermoplastic water-absorbent resin 23 can achieve a thickness in the above range that cannot be achieved by a fiber assembly having voids between fibers known in the art.

For example, in a polymer sheet known in the art in which a water-absorbent polymer is supported on a fiber sheet, such as a nonwoven fabric, the thickness obtained by the following measurement method is approximately 600 µm, whereas the thickness of the water-retention layer 2 in the present invention can be significantly reduced.

In addition, from the viewpoint of ensuring the water absorption capacity, the thickness of the water-retention layer 2 is preferably 140 µm or more, more preferably 150 µm or more, and even more preferably 200 µm or more.

### Method for Measuring Thickness of Water-Retention Layer 2

### (i) Pretreatment

After 5 minutes have elapsed from the start of the exothermic reaction of the warming device 10 to be measured, such as by opening the packaging bag, the central portion of the warming device is cut with scissors to prepare a cross section.

### (ii) Measurement

Next, in the cross section, a total of the following five locations are observed and imaged: the widthwise center portion of the water-retention layer 2 and locations spaced apart from the center portion by 10 mm and 20 mm to the left and right. The thickness of the water-retention layer 2 is measured from the captured image. The thickness is the average value of the five locations.

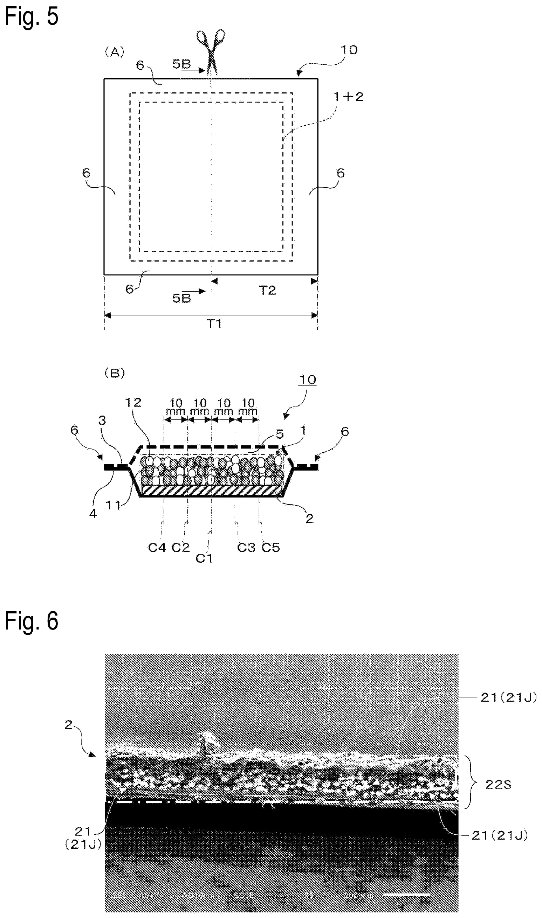

For example, as illustrated in Fig. 5(A), a cross section is prepared at a position corresponding to half the width T1 of the warming portion 10 (T1/2 = T2). Furthermore, as illustrated in Fig. 5(B), in the cross section, the thicknesses at the following locations are measured as described above: widthwise center portion C1 of the water retention part 2, locations C2 and C3 each spaced apart by 10 mm from center portion C1, and locations C4 and C5 each spaced apart by 20 mm from the center portion. The average is defined as the thickness of the water-retention layer 2. Although Fig. 5(B) illustrates the case of the laminated form illustrated in Fig. 1, the laminated form illustrated in Fig. 2 can also be measured in the same manner.

In the warming device 10 of the present embodiment, in the case where the water-retention layer 2 includes the dispersion layer 22S described above, examples of a preferred configuration of the dispersion layer 22S include the following configurations (1) to (10). The water-retention layer 2 may include any one of the following configurations (1) to (10) or may include two or more in combination.
(1) From the viewpoint of further increasing the absorption capacity of the water-retention layer 2 to more suitably decrease the excess water of the heat-generating layer 1 and to allow the heat-generating layer 1 to more favorably exhibit the excellent heat generation characteristics, a content proportion of the water-absorbent polymer 21 relative to a mass of the entire water-retention layer 2 is preferably 30 mass% or more, more preferably 40 mass% or more, and even more preferably 45 mass% or more.
   In addition, from the viewpoint of adjusting the water absorption capacity of the water-retention layer 2 to a more appropriate range to more suitably retain the water content required for heat generation of the heat-generating layer 1 and from the idea of appropriately maintaining the fluidity of the slurry in the example of the manufacturing method described later to increase the uniform dispersibility of the water-absorbent polymer, the content proportion of the water-absorbent polymer 21 relative to the mass of the entire water-retention layer 2 is preferably 65 mass% or less, more preferably 60 mass% or less, and even more preferably 55 mass% or less.
(2) From the viewpoint of making it easier to more uniformly arrange the water-absorbent polymer 21 in the dispersion layer 22S, a content proportion of the thermoplastic resin 22 relative to the mass of the entire water-retention layer 2 is preferably 20 mass% or more, more preferably 30 mass% or more, and even more preferably 40 mass% or more. This further improves the coatability (the property of being able to smoothly apply without unevenness and dripping) of the "slurry of the molten thermoplastic resin containing the water-absorbent polymer" in the example of the manufacturing method described later.

In addition, the content proportion of the thermoplastic resin 22 relative to the mass of the entire water-retention layer 2 is practically 60 mass% or less.

### Method for Measuring Content Mass Proportion of Water-Absorbent Polymer 21 and Thermoplastic Resin 22 Relative to Mass of Entire Water-Retention Layer 2

### (i) Pretreatment

The same treatment as (i) Pretreatment in the "Method for Measuring Proportion of Plan View Area of Fiber Material Relative to Plan View Area of Entire Water-Retention Layer 2" is performed.

### (ii) Measurement

Next, the water-retention layer 2 (dispersion layer 22S) and the sheet (the second sheet 4 in the form illustrated in Fig. 1 or the first sheet 3 in the laminated form illustrated in Fig. 2) covering its outer surface are taken out, and mass A is measured.

Thereafter, the water-retention layer 2 and the sheet taken out are immersed in deionized water to allow the water-absorbent polymer 21 to sufficiently absorb water. When the water-absorbent polymer 21 sufficiently absorbs water, the thermoplastic resin 22 and the water-absorbent polymer 21 are separated from each other. In addition, the sheet is also separated from the water-retention layer 2.

If the water-absorbent polymer 21 penetrates into the thermoplastic resin 22, the water-absorbent polymer 21 needs to be removed. For this purpose, the water-absorbent polymer 21 is squeezed out by applying pressure with the fingers. Whether the water-absorbent polymer 21 is sufficiently separated can be determined by whether the water-absorbent polymer 21 is interposed when the thermoplastic resin 22 is taken out and immersed in deionized water again.

Next, the thermoplastic resin 22 and the sheet in a state separated from the water-absorbent polymer 21 are taken out. These are allowed to stand under a Celsius temperature of 25°C, a pressure of 101.3 kPa, and a humidity of 50% for 72 hours to dry, and mass B of the thermoplastic resin 22 and mass C of the sheet are measured. Next, the water-absorbent polymer 21 in a water-absorbed state is allowed to stand under a Celsius temperature of 60°C, a pressure of 0.4 MPa, and a humidity of 50% for 72 hours to dry, and mass D is measured. From the mass ratio, the mass% (B/(A-C) = content mass proportion of the thermoplastic resin 22, D/(A-C) = content mass proportion of the water-absorbent polymer 21) are calculated.

(3) Sections each including a thickness cross-section cut out from the water-retention layer 2 with a width of 1 mm in a planar direction will be described. From the viewpoint of more favorably exhibiting the heat generation characteristics of the warming device 10 through the uniform water absorption capacity of the water-retention layer 2, a standard deviation of area values of the water-absorbent polymer 21 present in the thickness cut surfaces of the five sections is preferably 0 or more and 10 or less, more preferably 5 or less, and even more preferably 3 or less.

An example of such an arrangement configuration is illustrated in Fig. 6. In the example of the cross section illustrated in Fig. 6, the standard deviation of the area values of the water-absorbent polymer 21 indicated in white is 2.3, and it can be said that the water-absorbent polymer is uniformly arranged in a planar direction.

### Method for Measuring Standard Deviation of Area Values of Water-Absorbent Polymer 21 in Water-Retention Layer 2

### (i) Pretreatment

After 6 hours have elapsed since the start of the exothermic reaction of the warming device 10 to be measured, such as by opening the packaging bag, a cross section in the thickness direction of the warming device 10 is cut out in the same manner as in (i) Pretreatment in the "Method for Measuring Thickness of Water-Retention Layer 2" described above.

Here, "6 hours since the start of the exothermic reaction" means the time required for the water-absorbent polymer 21 of the water-retention layer 2 to reach a state where water has been released. Whether water has been sufficiently released from the water-absorbent polymer 21 can be determined by a change in the mass of the water-retention layer. When water has not been sufficiently released even after 6 hours have elapsed, the mass change is measured after another 6 hours have elapsed since that time. The mass change is measured from the difference between X and Y, where X is the mass at the start of measurement, that is, at the time when 6 hours have elapsed as described above, and Y is the mass after 1 hour has elapsed since that time. When the difference between X and Y is less than 1%, water is determined to have been sufficiently released from the water-absorbent polymer 21. The mass change at this time is measured using a mass meter capable of measuring to the third decimal place. The purpose of the above-described 6-hour period, as well as the determination of water release and the treatment method, all apply in the same way to other measurement methods described herein where the pretreatment requires the 6-hour exothermic reaction.

### (ii) Measurement

Next, in the cross-section, sections each including a thickness cross-section cut out with a width of 1 mm in the planar direction are prepared. Specifically, the sections are prepared at a total of five locations: the widthwise center portion of the water-retention layer 2, and locations spaced apart from the center portion by 10 mm and 20 mm to the left and right.

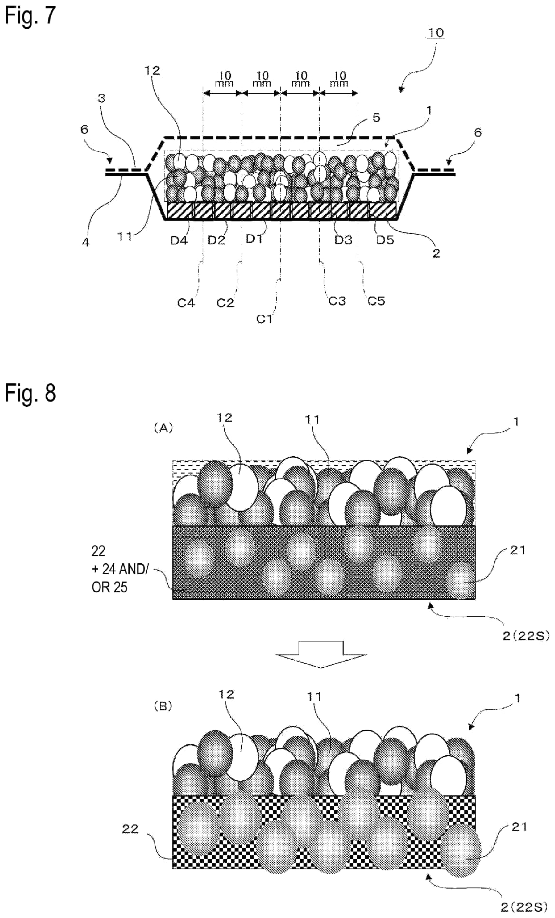

For example, as illustrated in Fig. 7, in the cross section of the warming device 10, sections D1 to D5 are prepared at a total of five locations C1 to C5: the center portion and two locations each to the left and right from the center portion. Although Fig. 7 illustrates the case of the laminated form illustrated in Fig. 1, the laminated form illustrated in Fig. 2 can also be measured in the same manner.

Next, the cross sections of the five locations are observed and imaged using a scanning electron microscope (SEM: JSM-6510, available from JEOL Ltd.).

Then, area ratios of the water-absorbent polymer 21 to others are calculated using image editing software. The area is the average value of the five locations. The area is measured across the entire width of 1 mm.

Also in this measurement, the image editing software described above is used.

(4) The dispersion layer 22S described above preferably contains one, or two or more selected from a water-soluble resin 24 and sodium chloride 25. The water-soluble resin 24 and the sodium chloride 25 are each brought into contact with water and dissolved, thereby forming voids in the dispersion layer 22S of the water-retention layer 2. The formation of the voids further increases the chance of contact between the water-absorbent polymer 21 and water and further improves the water absorption capacity of the water-retention layer 2. The one, or two or more selected from the water-soluble resin 24 and sodium chloride 25 described above are preferably mixed, in an example of a manufacturing method described later, in a tank in which the thermoplastic resin 22 in which the water-absorbent polymer 21 is dispersed is mixed. This enables suitable dispersed arrangement of one, or two or more selected from the water-soluble resin 24 and the sodium chloride 25 in the dispersion layer 22S.

From the viewpoint of more effectively exhibiting the action, a total content proportion of the water-soluble resin 24 and the sodium chloride 25 relative to the mass of the entire water-retention layer 2 is preferably 2 mass% or more and 30 mass% or less, and more preferably 25 mass% or less.

The water-soluble resin 24 is preferably present in a state of being mixed with the thermoplastic resin 22 at a stage before being brought into contact with a heat-generating layer raw material in the manufacturing process. This allows the water-soluble resin 24 to dissolve in water and to form a network of the thermoplastic resin 24 when the water-soluble resin 24 is brought into contact with water in the subsequent step of bringing the water-soluble resin 24 into contact with the heat-generating layer raw material. That is, the water-soluble resin 24 falls off and a network of the thermoplastic resin 22 is formed so as to surround the water-soluble resin 24, and a water channel is formed in voids S from which the water-soluble resin 24 has fallen off.

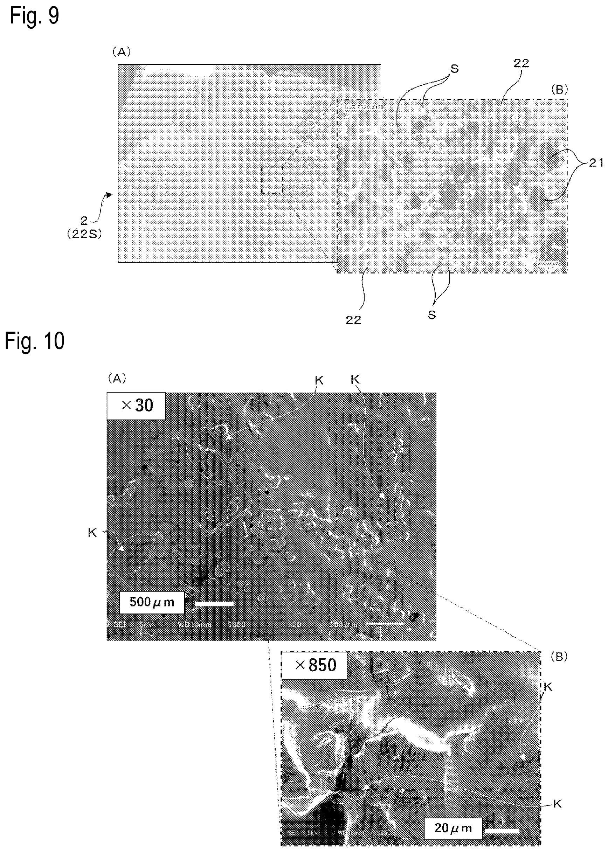

Examples of this configuration are illustrated in Figs. 8(A) and 8(B), and Fig. 9. In a schematic cross-sectional view of Fig. 8(A), the thermoplastic resin 22 and the water-soluble resin 24 are mixed to form the dispersion layer 22S, and the water-absorbent polymer 21 is dispersedly arranged and fixed in the dispersion layer 22S.

In the water-retention layer 2 adjacent to the heat-generating layer 1, the water-soluble resin 24 dissolves in water, thereby forming voids S in the portion of the water-soluble resin 24 as illustrated in Fig. 8(B). In this respect, for example, in observation of the water-retention layer 2 from the side of the heat-generating layer 1 illustrated in Figs. 9(A) and 9(B), it can be seen that the thermoplastic resin 22 incorporating the water-absorbent polymer 21 forms a network so as to surround voids S.

Voids S thus function as water channels in the network of the thermoplastic resin 22, and the water in the heat-generating layer 1 is more quickly supplied to the water-absorbent polymer 21 through the water channels and is retained.

The sodium chloride 25 is preferably present as particles in the layer of the thermoplastic resin before being brought into contact with water, that is, before being brought into contact with a heat-generating layer raw material in the manufacturing process. Thereafter, when the sodium chloride 25 is brought into contact with water in the step of being brought into contact with a heat-generating layer raw material, the particles of the sodium chloride 25 dissolve in water, thereby forming voids corresponding to the volume of the particles and forming water channels similar to those described above.

(5) The dispersion layer 22S preferably contains a surfactant. The surfactant increases the permeability of water in the water-retention layer 2. This further increases the chance of contact between the water-absorbent polymer and water, and further improves the water absorption capacity of the water-retention layer 2.

From the viewpoint of more effectively exhibiting the action, a content proportion of the surfactant relative to the mass of the entire water-retention layer 2 is preferably 0.1 mass% or more and 3 mass% or less, and more preferably 1 mass% or less.

### Method for Confirming Presence of Water-Soluble Resin 24, Sodium Chloride 25, and Surfactant in Water-Retention Layer 2

The water-retention layer 2 and the sheet covering the outer surface of the water-retention layer 2 that have been taken out are immersed in deionized water by the same method as the "Method for Measuring Content Mass Proportion of Water-Absorbent Polymer 21 and Thermoplastic Resin 22 Relative to Mass of Entire Water-Retention Layer 2" described above. Next, the thermoplastic resin 22, the water-absorbent polymer 21, and the sheet, as well as the water layer in which the water-retention layer 2 has been immersed are extracted.

Then, the thermoplastic resin 22 and the sheet that have been extracted are allowed to stand under a Celsius temperature of 25°C, a pressure of 101.3 kPa, and a humidity of 50% for 72 hours to dry. In addition, the extracted water layer is allowed to stand under a Celsius temperature of 60°C, a pressure of 0.4 MPa, and a humidity of 50% for 72 hours to dry. The precipitate after drying is analyzed. Specifically, the water-soluble resin is determined to be incorporated when the viscosity of the precipitate increases when the precipitate is immersed in deionized water with an equivalent mass to that of the precipitate. The sodium chloride is determined to be incorporated when the electrical conductivity of the precipitate increases when the precipitate is immersed in deionized water with an equivalent mass to that of the precipitate. Whether the surfactant is incorporated is determined by measuring according to the method described in "Kaimenkasseizai no Teisei Shiken Hoho (Qualitative Testing Method for Surfactant)" of Japan Spinners Inspecting Foundation (Tadanori Inoko, Takashi Nakabayashi, "Kaimenkasseizai no Teisei Shiken Hoho (Qualitative Testing Method for Surfactant)", Journal of the Japan Research Association for Textile End-Use, The Japan Research Association for Textile End-Uses, 1978, Vol. 19, No. 3, p. 96-103). This method enables determination of whether the water-soluble resin 24, the sodium chloride 25, and the surfactant are incorporated in the water-retention layer.

Furthermore, the content proportions of the water-soluble resin 24, the sodium chloride 25, and the surfactant relative to the entire water-retention layer 2 are calculated by dividing the respective masses of the precipitated water-soluble resin 24, sodium chloride 25, and surfactant by the mass of the entire water-retention layer 2 obtained by measuring in advance before the immersion described above. Specifically, the content proportions are calculated based on the calculation equation presented in the "Method for Measuring Content Mass Proportion of Water-Absorbent Polymer 21 and Thermoplastic Resin 22 Relative to Mass of Entire Water-Retention Layer 2" described above.

(6) The dispersion layer 22S preferably includes an arrangement configuration in which the adjacent water-absorbent polymers 21 are in contact with each other. This makes it easier for water to physically move between the water-absorbent polymers 21 in the water-retention layer 2, thereby further improving the water absorption capacity of the water-retention layer 2. An example of such an arrangement configuration is illustrated in Fig. 6. In the cross section illustrated in Fig. 6, a plurality of arrangement configurations 21J, in which the particles (white) of the water-absorbent polymer 21 are in contact with each other in a planar direction, is disposed in the thickness direction within the water-retention layer 2.

### Method for Confirming Arrangement Configuration in which Water-Absorbent Polymers Are in Contact with Each Other

After 6 hours have elapsed since the start of the exothermic reaction of the warming device 10 to be measured, such as by opening the packaging bag, a cross section in the thickness direction of the warming device 10 is cut out in the same manner as in (i) Pretreatment in the "Method for Measuring Thickness of Water-Retention Layer 2" described above. At this time, sections each including a thickness cross-section cut out with a width of 1 mm in the planar direction are prepared by the same method as the "Method for Measuring Standard Deviation of Area Values of Water-Absorbent Polymer 21 in Water-Retention Layer 2". Specifically, in the cross section, the sections are prepared at a total of five locations: the widthwise center portion of the water-retention layer 2, and locations spaced apart from the center portion by 10 mm and 20 mm to the left and right (e.g., sections D1 to D5 in Fig. 7).

Next, the thickness cross sections of the five locations are confirmed using an SEM. When the water-absorbent polymers (white) are in a state of being adjacent to each other as illustrated in Fig. 6 in the visual confirmation of the observed cut surface, the water-absorbent polymers are naturally determined to be in a state of being adjacent to each other even when the water-absorbent polymers are swollen by absorbing water, that is, even in a state before the start of the reaction.

(7) In the configuration of (6), the water-absorbent polymers 21 preferably contain polymers exposed to a surface of the dispersion layer 22S, the surface on the side of the heat-generating layer 1. This exposure serves as a driving force, further facilitating the intake of water from the heat-generating layer 1.

(8) The dispersion layer 22S described above preferably includes a cracked portion K on the surface on the side of the heat-generating layer 1. This makes it easier for water to permeate through the cracked portion K, further increasing the chance of contact between the water-absorbent polymer 21 and water and further improving the water absorption capacity of the water-retention layer 2. Examples of the cracked portions K are illustrated in regions indicated by one-dot chain lines and arrows in Figs. 10(A) and 10(B).

### Method for Confirming Exposure of Water-Absorbent Polymer and Cracks on Heat-Generating Layer 1-Side Surface of Dispersion Layer 22S

After 6 hours have elapsed since the start of the exothermic reaction of the warming device 10 to be measured, such as by opening the packaging bag, the heat-generating layer 1 and the sheet (the first sheet 3 in the laminated form illustrated in Fig. 1 or the second sheet 4 in the laminated form illustrated in Fig. 2) covering its outer surface are removed by the same method as the "Method for Measuring Proportion of Plan View Area of Fiber Material Relative to Plan View Area of Entire Water-Retention Layer 2" described above.

Next, the surface of the dispersion layer 22S is observed using an SEM. In the visual confirmation of the observed surface, when the water-absorbent polymer 21 or cracks as illustrated in Fig. 10 are confirmed, the water-absorbent polymer 21 is determined to be exposed or to have cracks.

The presence or absence of cracks remains unchanged between immediately after opening and after 6 hours have elapsed.

(9) In the water-retention layer 2, the dispersed bodies of water are preferably uniformly dispersed in the thickness direction and a planar direction. This is presumed from the uniform dispersion of the water-absorbent polymer 21 in the thickness direction and a planar direction. The supply of water becomes more significant by any one or a plurality of actions of the configurations (4) to (8).

(10) An average particle diameter of the water-absorbent polymer in the water-retention layer is preferably 10 µm or more from the viewpoint of handleability.

In addition, from the viewpoint of reducing the thickness of the warming device, the average particle diameter of the water-absorbent polymer in the water-retention layer is preferably 320 µm or less, more preferably 180 µm or less, and even more preferably 80 µm or less.

The thickness of the warming device 10 of the present embodiment is practically 0.5 mm or more.

In addition, from the viewpoint of reducing the thickness, the thickness of the warming device 10 is preferably 1.2 mm or less, more preferably 1.0 mm or less, and even more preferably 0.8 mm or less. The thickness of the warming device 10 is measured using a constant-pressure thickness gauge PG-20J (available from Teclock Co., Ltd.) in accordance with JIS K 6250.

As described above, the warming device 10 of the present embodiment can achieve good heat generation characteristics while achieving thickness reduction.

From this viewpoint, a maximum temperature of the warming device 10 during heat generation is preferably 45°C or higher, more preferably 60°C or higher, and even more preferably 65°C or higher. This temperature characteristic allows a user to feel an appropriate sense of warmth.

In addition, from the viewpoint of ensuring safety, the maximum temperature of the warming device 10 during heat generation is preferably 75°C or lower, more preferably 73°C or lower, and even more preferably 70°C or lower.

Furthermore, from the viewpoint of allowing a user to feel the bodily sensation of heat generation earlier to further improve the feeling of use, the time for the warming device 10 to reach 45°C from 35°C is preferably more than 0 minutes. The time is practically preferably 5 minutes or less, more preferably 3 minutes or less, and even more preferably 1 minute or less.

### Method for Measuring Heat Generation Characteristics of Warming Device 10

The heat generation is measured using a measuring machine in accordance with JIS S4100 by attaching the second sheet 4 side of the warming device 10 to the measuring surface within one minute after opening.

In the warming device 10 of the present embodiment, the oxidizable metal 11 and the carbon component 12 constituting the heat-generating layer 1, and the water-absorbent polymer 21, the thermoplastic resin 22, the thermoplastic water-absorbent resin 23, the water-soluble resin 24, and the surfactant constituting the water-retention layer 2 can be made of any of various materials that can favorably achieve the effects described above.

For the oxidizable metal 11, any of various metals that generate oxidation reaction heat can be used; examples include one, or two or more powders and fibers selected from the group consisting of iron, aluminum, zinc, manganese, magnesium, and calcium. Among these, iron powder is preferably contained from the viewpoint of handleability, safety, manufacturing cost, storability, and stability.

Examples of the iron powder include one, or two or more selected from the group consisting of reduced iron powder and atomized iron powder.

For the carbon component 12, any of various carbon components having one, or two or more selected from water retention capacity, oxygen supply capacity, and catalytic capacity can be used. For example, one, or two or more selected from activated carbon, acetylene black, and graphite can be used. Among these, activated carbon is preferably used from the viewpoints of facilitating adsorption of oxygen in a wet state and making it easier to maintain water content in the heat-generating layer 1.

More preferably, for the activated carbon, one, or two or more of fine powdery materials or small-granular materials selected from coconut shell charcoal, wood powder charcoal, and peat charcoal are used. Among these, wood powder charcoal is preferred.

The water-absorbent polymer 21 in the water-retention layer 2 typically contains a hydrophilic polymer having a crosslinked structure capable of absorbing and retaining a liquid in an amount of 20 times or more the weight of the polymer itself. The shape of the water-absorbent polymer 21 preferably includes one, or two or more shapes selected from the group consisting of a granular shape, a spherical shape, a lump shape, a grape cluster shape, and a fibrous shape.

The water-absorbent polymer 21 preferably contains one, or two or more selected from the group consisting, for example, of starch, crosslinked carboxymethylated cellulose, a polymer of acrylic acid or an alkali metal salt of acrylic acid or a copolymer of these, and polyacrylic acid, its salt, or a polyacrylic acid salt graft polymer. Among these, the water-absorbent polymer 21 preferably contains one, or two or more selected from a polymer of acrylic acid or an alkali metal salt of acrylic acid or a copolymer of these, and polyacrylic acid, its salt, or a polyacrylic acid salt graft polymer, because this makes it easier to maintain the amount of water carried by the water-absorbent polymer 21 within a specific range.

The thermoplastic resin 22 in the water-retention layer 2 is preferably a hot-melt material having fluidity at normal or high temperatures from the viewpoints of providing solidity to maintain the shape of the water-retention layer 2 before, during, and after use, and of more favorably forming the water-retention layer 2 in the coating method described later. For the material for forming the water-retention layer 2, a common hot-melt material can be used without any particular limitation.

Specific examples of the hot-melt material include styrene-isobutylene-based, acrylic-based, urethane-based, rubber-based, silicon-based, polyisobutylene-based, styrene-isobutylene-styrene-based, and polyacrylic acid-based polymers. Among these, a styrene-isobutylene-based polymer is preferred from the viewpoint of coatability.

From the viewpoints of providing solidity to maintain the shape of the water-retention layer 2 before, during, and after use, and of more favorably forming the water-retention layer 2 in the coating method described later, examples of the thermoplastic water-absorbent resin 23 in the water-retention layer 2 include materials having fluidity at normal or high temperatures and having water absorption capacity.

Specific examples of the thermoplastic water-absorbent resin 23 include nonionic polyalkylene oxide-based and nylon-based materials.

Examples of the water-soluble resin 24 contained in the water-retention layer 2 include materials having the same characteristics as those of the thermoplastic resin 22 and having water-soluble characteristics.

Specific examples of the water-soluble resin material include materials having a hydrophilic group, such as polyethylene oxide-based and polyethylene glycol-based materials. Among these, a polyethylene oxide-based material is preferred from the viewpoint of coatability.

For the surfactant contained in the water-retention layer 2, a common surfactant can be used without any particular limitation to assist the water absorption of the water-absorbent polymer.

Specific examples of the surfactant include nonionic surfactants, such as polyoxyethylene alkyl ethers, polyoxyethylene alkyl phenyl ethers, and polyoxyethylene polyoxypropylene glycol. Among these, a polyoxyethylene alkyl ether is preferred from the viewpoint of the heat-generating performance of the coating material.

For the first sheet 3, any of various materials capable of serving as an air-permeable member can be used. Examples include various fiber sheets, such as nonwoven fabrics, and porous sheets made of a synthetic resin.

Specifically, the nonwoven fabric preferably includes one, or two or more selected, for example, from needle-punched nonwoven fabrics, air-through nonwoven fabrics, and spunbond nonwoven fabrics.

The porous sheet made of a synthetic resin preferably includes, for example, a film obtained by incorporating calcium carbonate or the like into polyethylene or polypropylene and stretching the mixture. In the case of using such a porous sheet, a fiber sheet, such as the nonwoven fabric described above may be laminated on the outer surface of the porous sheet to enhance the texture of the first sheet 3.

In addition, a sheet other than a porous sheet may be used as long as it is impermeable to water but permeable to oxygen and water vapor, and leakage prevention is ensured. For example, a woven fabric, a nonwoven fabric, paper, synthetic paper, or the like obtained by mixing one, or two or more selected from artificial fibers, such as polyamide, vinylon, polyester, rayon, acetate, acrylic resin, polyethylene, polypropylene, and polyvinyl chloride, and natural fibers, such as pulp, cotton, hemp, silk, and animal hair, can be used as the first sheet 3.

The second sheet 4 is preferably less air-permeable than the first sheet from the viewpoint of directing the warmth. Examples include films made of the synthetic resin described above but having no holes or having a smaller number of holes than the porous sheet of the first sheet 3. In addition, a fiber sheet, such as the nonwoven fabric described above, may be laminated on the outer surface of the film made of the synthetic resin described above to enhance the texture.

The warming device 10 of the present embodiment may further contain another agent together with the agent described above within a range not inhibiting the effects described above. For example, the warming device 10 may contain an agent that affects the feeling of use, such as menthol or a fragrance.

Next, a preferred embodiment of the method for manufacturing the warming device of the present invention will be described below.

The method for manufacturing the warming device of the present embodiment (hereinafter also referred to simply as the manufacturing method of the present embodiment or the manufacturing method) preferably includes (S1) applying a coating material containing the oxidizable metal 11, the carbon component 12, and water (also collectively referred to as a heat-generating layer raw material G1) onto one surface of a first raw material sheet Q1. Step S1 can form the heat-generating layer 1 in the warming device 10 described above.

Next, the method preferably includes (S2) applying: a slurry of a molten thermoplastic resin 22 containing the water-absorbent polymer 21; and/or a molten thermoplastic water-absorbent resin 23 (also collectively referred to as a water-retention layer raw material G2) onto one surface of a second raw material sheet Q2. Step S2 can form the water-retention layer 2 in the warming device 10 described above. At this time, the molten thermoplastic resin 22 containing the water-absorbent polymer 21 is a slurry, thus enabling suitable control of the dispersibility of the water-absorbent polymer 21 by stirring or the like. This control can suitably achieve the dispersed arrangement of the water-absorbent polymer 21 in the water-retention layer 2 and uniform water absorption capacity in the water-retention layer 2 in the warming device 10 described above.

Furthermore, the method preferably includes (S3) bonding the first raw material sheet Q1 and the second raw material sheet Q2 in such a way that their coated surfaces face each other. Step S3 can form a laminated structure in which the heat-generating layer 1 and the water-retention layer 2 are disposed in the space sandwiched between the first sheet 3 and the second sheet 4 in the warming device 10.

One or both selected from the first raw material sheet Q1 and the second raw material sheet Q2 used in steps S1, S2, and S3 are preferably air-permeable sheets. That is, either the first raw material sheet Q1 or the second raw material sheet Q2 serves as the air-permeable first sheet 3 in the warming device 10 described above.

In the case where the first raw material sheet Q1 is the air-permeable first sheet 3, for example, a laminated structure of the warming device 10 as illustrated in Fig. 1 can be formed.

On the other hand, in the case where the second raw material sheet Q2 is the air-permeable first sheet 3, for example, a laminated structure of the warming device 10 as illustrated in Fig. 2(A) and/or Fig. 2(B) can be formed by making the area of the water-retention layer 2 smaller than the area of the heat-generating layer 1.

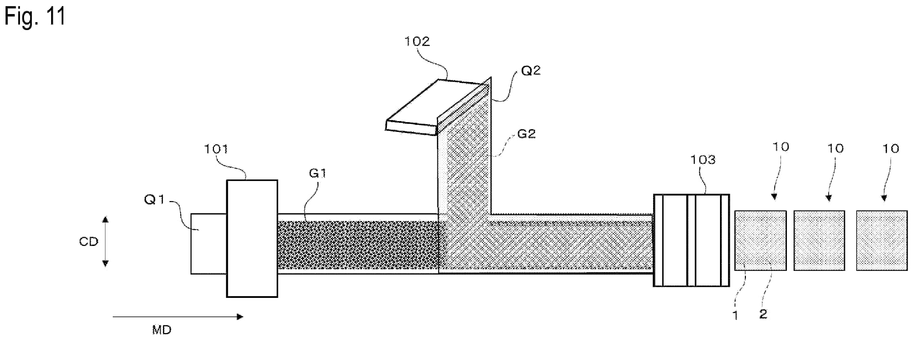

An example of an embodiment of this manufacturing method is illustrated in Fig. 11.

In the example of the manufacturing method illustrated in Fig. 11, steps S1 and S2 are continuously performed using a long first raw material sheet Q1 and a long second raw material sheet Q2; step S1 is carried out in a heat-generating layer coating device 101, and step S2 in a water-retention layer coating device 102. Step S3 is performed continuously downstream of steps S1 and S2. More specifically, in step S3, the first raw material sheet Q1 and the second raw material sheet Q2 are overlapped with their coated surfaces facing each other downstream of steps S1 and S2, and a laminate is continuously formed along a machine direction (MD). In a sealing and cutting device 103 further downstream, the laminate is cut at predetermined length intervals along a cross direction (CD) perpendicular to the machine direction, and at the same time, the four sides of each cut section are sealed. A plurality of the warming devices 10 of the present embodiment described above can be manufactured along the MD direction in this manner.

In step S2, the water-retention layer raw material G2 to be applied is preferably controlled as follows.

### (S21) Temperature Control of Melting Tank for Thermoplastic Resin and Thermoplastic Water-Absorbent Resin

The melting tank is preferably controlled at a temperature higher than the softening points of the thermoplastic resin and the thermoplastic water-absorbent resin.

The melting tank is preferably controlled at a temperature lower than the carbonization points of the thermoplastic resin and the thermoplastic water-absorbent resin.

### (S22) Nozzle Lip Clearance Control

The nozzle for applying the water-retention layer raw material G2 is preferably controlled with a lip clearance wider than the maximum particle diameter of the water-absorbent polymer 21.

In the cutting along the CD direction and the sealing of the four sides of the cut section in the sealing and cutting device 103, steps S1 and S2 are preferably performed as follows from the viewpoints of further enhancing the sealing properties of the resulting warming device 10 and of preventing contamination of the sealing and cutting device 103.

That is, the coating material and either the slurry or the molten thermoplastic water-absorbent resin, or both the slurry and the molten thermoplastic water-absorbent resin, are preferably applied intermittently in the MD direction.

Moreover, the first raw material sheet Q1 and the second raw material sheet Q2 outside the coated portion in the MD direction are preferably sealed.

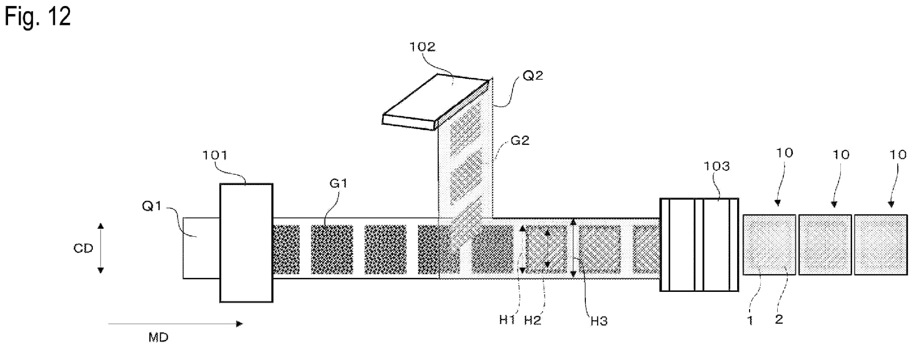

An example of an embodiment of this manufacturing method is illustrated in Fig. 12.

Coating widths (H1, H2) in the CD direction of the coating material and either the slurry or the molten thermoplastic water-absorbent resin, or both the slurry and the molten thermoplastic water-absorbent resin, are preferably made narrower than width (H3) of the first raw material sheet Q1 and the second raw material sheet Q2.

Moreover, the first raw material sheet Q1 and the second raw material sheet Q2 outside the coated portion in the CD direction are preferably sealed.

In addition, from the viewpoint of preventing interference with the air permeability of the warming device 10 to be manufactured, the coating widths (H1, H2) described above preferably satisfy H1 > H2.

An example of an embodiment of these manufacturing methods is illustrated in Fig. 12.

From the viewpoint of maintaining good air permeability of the warming device 10 to be manufactured, the coating material and either the slurry or the molten thermoplastic water-absorbent resin, or both the slurry and the molten thermoplastic water-absorbent resin, are preferably applied intermittently in the CD direction. This is particularly effective in the case where the second raw material sheet Q2 is an air-permeable sheet and the laminated structure of the warming device 10 as illustrated in Fig. 2 is manufactured.

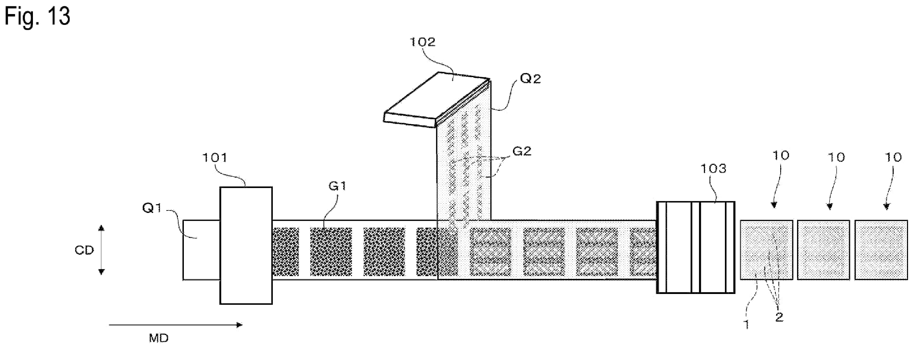

An example of an embodiment of this manufacturing method is illustrated in Fig. 13.

In the case where step S2 includes applying the slurry onto one surface of the second raw material sheet Q2, an average particle diameter of the water-absorbent polymer 21 is, from the viewpoint of handleability, preferably 1 µm or more, more preferably 10 µm or more, and even more preferably 40 µm or more.

In addition, the average particle diameter of the water-absorbent polymer 21 is, from the viewpoint of further enhancing the dispersibility of the water-absorbent polymer 21 and the coatability of the slurry, preferably 360 µm or less, more preferably 100 µm or less, and even more preferably 80 µm or less.

In step S1, sodium chloride may be incorporated into the coating material. This can eliminate the need for incorporating sodium chloride later, efficiently improving the heat generation characteristics of the heat-generating layer 1.

From the viewpoint of ensuring coatability, the content proportion of sodium chloride in the coating material is preferably 0 mass% or more and 30 mass% or less, more preferably 17 mass% or less, and even more preferably 10 mass% or less.

In the case where step S2 includes applying the slurry onto one surface of the second raw material sheet Q2, from the viewpoint of further improving the coatability, the content proportion of the thermoplastic resin 22 in the slurry is preferably set similarly to the "content proportion of the thermoplastic resin 22 relative to the mass of the entire water-retention layer 2" described above.

In the case where step S2 includes applying the slurry onto one surface of the second raw material sheet Q2, one, or two or more selected from the water-soluble resin 24 and the sodium chloride 25 are preferably incorporated into the slurry. This allows the water-soluble resin 24 and the sodium chloride 25 to dissolve in water to form water channels in the water-retention layer 2 (dispersion layer 22S) of the resulting warming device 10 as described above. This in turn allows water in the heat-generating layer 1 to be more rapidly supplied to the water-absorbent polymer 21 and retained in the polymer.

In this respect, in a step prior to step S2 and/or in step S2, a treatment of mixing one or two selected from the water-soluble resin 24 and the sodium chloride 25 is preferably performed in a tank in which the thermoplastic resin 22 in which the water-absorbent polymer 21 is dispersed is mixed. This improves the dispersion state of the water-soluble resin 24 and the sodium chloride 25 in the slurry, enabling the water channels to be formed more uniformly with less deviation in the water-retention layer 2 (dispersion layer 22S).

From the viewpoint of more effectively exhibiting the action, the total content proportion of the water-soluble resin 24 and the sodium chloride 25 relative to the mass of the entire slurry is preferably set similarly to the "total content proportion of the water-soluble resin 24 and the sodium chloride 25 relative to the mass of the entire water-retention layer 2" described above.

From the same viewpoint as described above, it is preferred to incorporate a surfactant into the slurry, and it is more preferred to perform this treatment in a tank in which the thermoplastic resin 22 in which the water-absorbent polymer 21 is dispersed is mixed.

The content proportion of the surfactant relative to the mass of the entire slurry is preferably set similar to the "content proportion of the surfactant relative to the mass of the entire water-retention layer 2" described above.

In the case where step S2 includes applying the slurry onto one surface of the second raw material sheet Q2, the method preferably includes pressing the applied slurry with a roll. This makes it possible to more favorably form the above-described cracks in the water-retention layer 2. In particular, it is preferred to incorporate one, or two or more selected from the water-soluble resin 24 and the sodium chloride 25 into the slurry and apply and press the mixture, because the water-soluble resin 24 and the sodium chloride 25 serve as starting points to facilitate clearer formation of cracks.

From the viewpoint of more clearly generating cracks in the water-retention layer 2, the pressing force is preferably 0.2 kN or more, more preferably 5 kN or more, and even more preferably 30 kN or more.

The pressing force is preferably 94 kN or less from the viewpoint of ensuring water absorption performance.

In the case where step S2 includes applying the slurry onto one surface of the second raw material sheet Q2, from the viewpoint of further improving coatability, a ratio of a mass proportion of the thermoplastic resin 22 to a total mass proportion of the water-absorbent polymer 21, the sodium chloride 25, and the water-soluble resin 24 (mass proportion of the thermoplastic resin/(mass proportion of the water-absorbent polymer + mass proportion of the sodium chloride + mass proportion of the water-soluble resin)) accounting for a total mass of the slurry is preferably 0.5 or more, more preferably 0.8 or more, and even more preferably 1.3 or more.

From the viewpoint of further improving water absorption properties, the ratio of the mass proportion of the thermoplastic resin 22 to the total mass proportion of the water-absorbent polymer 21, the sodium chloride 25, and the water-soluble resin 24 (mass proportion of thermoplastic resin/(mass proportion of the water-absorbent polymer + mass proportion of the sodium chloride + mass proportion of the water-soluble resin)) accounting for the total mass of the slurry is preferably 5 or less, more preferably 3 or less, and even more preferably 2 or less.

### Examples

The present invention will be described in further detail below based on examples; however, the present invention is not to be construed as being limited by these. In the present examples, "parts" and "%" are all based on mass unless otherwise specified. In Table 1 below, "-" means that the corresponding value is not included in the item or the like.

### Example 1

A warming device sample of Example 1 illustrated in Fig. 1 was prepared by the manufacturing method illustrated in Fig. 11.

For the first sheet 3, an air-permeable sheet with an air permeability of 1500 sec/100 mL and a size of 63 mm × 63 mm was used. For the second sheet 4, a laminated paper with a basis weight of 32 g/m² and a size of 63 mm × 63 mm, obtained by laminating paper and film, was used.

The heat-generating layer 1 and the water-retention layer 2 each had a size of 49 mm × 49 mm and the composition presented in Table 1. The ratio of the area M1 of the heat-generating layer to the area M2 of the water-retention layer was 1.0.

Specifically, the water-retention layer 2 was formed as a thermoplastic resin layer (dispersion layer) (see Fig. 3(A)) in which a water-absorbent polymer was dispersed, by mixing under heating, 50 mass% of powder of a water-absorbent polymer "Sunfresh YH-2" (trade name, Sanyo Chemical Industries, Ltd., average particle diameter 65 mm) and 50 mass% of solid of a thermoplastic resin "H2052U" (trade name, available from Bostik-Nitta Co., Ltd.).

### Examples 2 and 3

Warming device samples of Examples 2 and 3 were prepared in the same manner as in Example 1, except that the heat-generating layer 1 and the water-retention layers 2 each had the composition presented in Table 1. More specifically, the water-retention layer 2 as illustrated in Fig. 3(A) was formed by incorporating sodium chloride particles into the same water-absorbent polymer and thermoplastic resin as in Example 1.

### Examples 4 and 5

Warming device samples of Examples 4 and 5 were prepared in the same manner as in Example 1, except that the water-retention layers 2 each had the composition presented in Table 1.

The water-retention layer 2 in Example 4 was formed using a thermoplastic resin "ME-716" (trade name, available from Henkel Japan Ltd.).

The water-retention layer 2 in Example 5 was formed as a layer (see Fig. 3(B)) of 100 mass% of a thermoplastic water-absorbent resin "Aquacoke TW" (trade name, available from Sumitomo Seika Chemicals Co., Ltd.).

### Examples 6 to 10

Warming device samples of Examples 6 to 10 were prepared in the same manner as in Example 1, except that the water-retention layers 2 each had the composition presented in Table 1.

The water-retention layers 2 of Examples 6 and 7 each had the composition presented in Table 1, by incorporating a powder of a water-soluble resin "ALKOX L-11" (trade name, available from Meisei Chemical Works, Ltd.) into the powder of the water-absorbent polymer and the solid of the thermoplastic resin in Example 1 and mixing under heating.

The water-retention layers 2 in Examples 8 and 9 were each formed to have the composition presented in Table 1 by incorporating a surfactant "1150S-60" (trade name, available from Kao Corporation) into the composition of Example 7 without incorporating the water-soluble resin.

The water-retention layer 2 in Example 10 was formed to have the composition presented in Table 1 by removing the water-soluble resin from the composition of Example 6. The arrangement of the water-retention layer 2 in Example 10 was a stripe form illustrated in Fig. 4(B), and "area of heat-generating layer/area of water-retention layer" was set to "2".

### Comparative Example 1

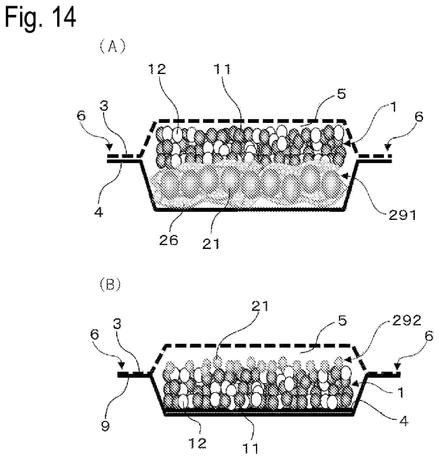

A warming device sample of Comparative Example 1 was prepared as presented in Table 1 in the same manner as in Example 1, except that a polymer sheet "PS-120PHC" (trade name, available from Inoshi Co., Ltd.) in which a water-absorbent polymer 21 was sandwiched between pulp 26 was used as a water-retention layer 291 instead of the water-retention layer 2 (Fig. 14(A)).

### Comparative Example 2

A warming device sample of Comparative Example 2 was prepared as presented in Table 1 in the same manner as in Example 1, except that the water-absorbent polymer (powder) used in Example 1 was arranged, in an amount equivalent to that in Example 1, between the heat-generating layer 1 and the first sheet 3; the second sheet 4 with an area (49 mm × 49 mm) equivalent to the plan view area of the heat-generating layer 1 was brought into contact with the heat-generating layer 1; and further the outside of the second sheet 4 was covered with a covering sheet 9 (the same material as the second sheet 4, basis weight 71 g/m², size 63 mm × 63 mm) (Fig. 14(B)). The powder water-absorbent polymer was sprayed on the uneven surface 1 of the heat-generating layer 1 and formed a water-retention layer 292 along the uneven surface of the heat-generating layer 1 as illustrated in Fig. 14(B). This made it impossible to take out the water-absorbent polymer to measure the layer thickness; thus, the item of "Thickness of water-retention layer" in Table 1 was indicated as "-".

### Comparative Example 3

A warming device sample of Comparative Example 3 was prepared in the same manner as in Comparative Example 2, except that the water-absorbent polymer "Sunfresh ST-D500" (trade name, available from Sanyo Chemical Industries, Ltd., average particle diameter 380 mm) (powder) presented in Table 1 was used in an amount equivalent to that in Example 1.

In each of the examples and comparative examples, each item relating to the water-retention layer was measured. At this time, the measurement method described above was used as necessary. Similarly, the thickness of the warming device was measured based on the measurement method described above.

Next, the maximum temperature generated by the warming device sample, the rise time (the time until reaching 45°C from 35°C), and the duration of heat generation (the time during which the temperature was 45°C or higher) were measured by the following methods.

In the heat generation measurement, the temperature was plotted against the time axis at 10-second intervals, with the time axis as the horizontal axis and the temperature as the vertical axis. In addition, from the plotted results, [maximum temperature (°C)], [rise time (min)], and [duration (min)] were determined. Specifically, [maximum temperature (°C)] was defined as the highest temperature (°C) from the start to the end of measurement, [rise time (min)] was defined as the time (min) required to reach 45°C from 35°C after the start of measurement, and [duration (min)] was defined as the time (min) during which the temperature was 45°C or higher from the start to the end of measurement.

Furthermore, the "coatability" in the manufacturing process of each warming device was evaluated by the following method.

Five evaluators familiar with sheet processing assessed the coatability of the water-retention layer with the naked eye and performed sensory evaluation based on the average of their assessments. Twenty (20) g of the sample was heated to 180°C and evaluated in the state after 20 minutes had elapsed. The sample of each example was scored on the basis that Sample 1 (only thermoplastic resin "H2052U") was scored 100 as having the best coatability and Sample 2 (Example 3) was scored 0.

The results of the evaluation measurement are presented in Table 5.

**[Table 1]**

| | | Example 1 | Example 2 | Example 3 | Example 4 | Example 5 | Example 6 | Example 7 | Example 8 | Example 9 | Example 10 | Comparative Example 1 | Comparative Example 2 | Comparative Example 3 |
|---|---|---|---|---|---|---|---|---|---|---|---|---|---|---|
| Heat-generating layer | Iron powder [mass%] | 55.07 | 57.95 | ← | 55.07 | ← | ← | ← | ← | ← | ← | ← | ← | ← |
| | Activated carbon [mass%] | 4.41 | 4.64 | ← | 4.41 | ← | ← | ← | ← | ← | ← | ← | ← | ← |
| | Xanthan gum [mass%] | 0.14 | ← | ← | ← | ← | ← | ← | ← | ← | ← | ← | ← | ← |
| | Tripotassium phosphate [mass%] | 0.99 | 1.04 | ← | 0.99 | ← | ← | ← | ← | ← | ← | ← | ← | ← |
| | 48% Aqueous potassium hydroxide solution [mass%] | 0.28 | 0.29 | ← | 0.28 | ← | ← | ← | ← | ← | ← | ← | ← | ← |
| | Sodium chloride [mass%] | 4.97 | 0.00 | ← | 4.97 | ← | ← | ← | ← | ← | ← | ← | ← | ← |
| | Deionized water [mass%] | 34.14 | 35.94 | ← | 34.14 | ← | ← | ← | ← | ← | ← | ← | ← | ← |
| Water-retention layer | Water-absorbent polymer Sunfresh YH-2 [mass%] | 50 | 40 | 50 | 50 | - | 40 | 30 | 39.2 | 39.8 | 50 | - | 100 | - |
| | Water-absorbent polymer Sunfresh ST-D500 [mass%] | - | - | - | - | - | - | - | - | - | - | - | - | 100 |
| | Thermoplastic resin H2052U [mass%] | 50 | 40 | 33.3 | - | - | 40 | 40 | 58.8 | 59.7 | 50 | - | - | - |
| | Thermoplastic resin ME-716 [mass%] | - | - | - | 50 | - | - | - | - | - | - | - | - | - |
| | Mass proportion of thermoplastic resin/(mass proportion of water-absorbent polymer + mass proportion of sodium chloride + mass proportion of water-soluble resin) [-] | 1.00 | 0.67 | 0.50 | 1.00 | - | 0.67 | 0.67 | 1.50 | 1.50 | 1.00 | - | - | - |
| | Thermoplastic water-absorbent resin [mass%] | - | - | - | - | 100 | - | - | - | - | - | - | - | - |
| | Water-soluble resin [mass%] | - | - | - | - | - | 20 | 30 | - | - | - | - | - | - |
| | Surfactant [mass%] | - | - | - | - | - | - | - | 2.0 | 0.5 | - | - | - | - |
| | Polymer sheet [mass%] | - | - | - | - | - | - | - | - | - | - | 100 | - | - |
| | Sodium chloride [mass%] in water-retention layer | - | 20.0 | 16.6 | - | - | - | - | - | - | - | - | - | - |
| | Proportion [%] of plan view area of fiber material relative to plan view area of entire water-retention layer | 0 | 0 | 0 | 0 | 0 | 0 | 0 | 0 | 0 | 0 | 75 | 0 | 0 |
| | Thickness [µm] of water-retention layer | 140 | 210 | 200 | 200 | 300 | 180 | 120 | 170 | 180 | 160 | 600 | - | - |
| | Content proportion [mass%] of water-absorbent polymer relative to mass of entire water-retention layer | 23 | 18 | 15 | 25 | - | 15 | 10 | 22 | 21 | 25 | - | - | - |
| | Content proportion [mass%] of thermoplastic resin relative to mass of entire water-retention layer | 46 | 38 | 33 | 49 | - | 38 | 36 | 52 | 51 | 46 | - | - | - |
| | Standard deviation [%] of water-absorbent polymer area value in water-retention layer | 2.3 | 2.5 | 1.6 | 1.9 | - | 4.6 | 5.2 | 4.3 | 2.6 | 2.3 | 26.8 | - | - |
| | Area of heat-generating layer/area of water-retention layer [-] | 1.00 | 1.00 | 1.00 | 1.00 | 1.00 | 1.00 | 1.00 | 1.00 | 1.00 | 2.00 | 1.00 | 1.00 | 0.35 |
| Evaluation results | Warming device thickness [mm] | 0.79 | 0.83 | 0.87 | 0.82 | 0.90 | 0.70 | 0.77 | 0.78 | 0.77 | 0.78 | 1.29 | 0.91 | 1.08 |
| | Maximum temperature [°C] | 67.8 | 70.7 | 72.5 | 63.3 | 59.2 | 66.6 | 52.6 | 61.0 | 56.3 | 61.2 | 64.9 | 63.2 | 63.7 |
| | Rise time [min] | 1.0 | 3.0 | 0.8 | 4.2 | 0.5 | 1.0 | 2.0 | 1.5 | 3.5 | 1.3 | 1.3 | 0.8 | 1.3 |
| | Duration of heat generation (time during which temperature was 45°C or higher) [mini | 20.7 | 29.5 | 22.2 | 28.2 | 27.2 | 29.3 | 17.8 | 36.2 | 42.5 | 35.8 | 35.2 | 22.8 | 27.0 |
| | Coatability of water-retention layer | 65 | 18 | 0 | 62 | 28 | 27 | 27 | 76 | 72 | 65 | - | - | - |

As presented in Table 1, the warming device sample of each example was able to achieve both thickness reduction and a high maximum temperature during heat generation compared with the warming device sample of each comparative example. Both the thickness reduction and the high maximum temperature were also achieved by increasing "the area of the heat-generating layer/the area of the water-retention layer" in Example 10.

In addition, the warming device sample of each example exhibited good heat generation characteristics equivalent to those of the warming device sample of each comparative example in terms of the rise time and the duration of heat generation despite having a smaller thickness than the warming device sample of each comparative example.

Furthermore, Examples 1, 4, and 8 to 10 indicated good results in terms of the coatability of the water-retention layer in the manufacturing process of the warming device sample of each example. This is due to the large value of the mass proportion of the thermoplastic resin/(the mass proportion of the water-absorbent polymer + the mass proportion of sodium chloride + the mass proportion of the water-soluble resin).

Although the present invention has been described in conjunction with its embodiments and examples, we do not intend to limit our invention in any of the details of the description unless otherwise specified, but rather, we believe that the invention should be broadly construed without departing from the spirit and scope of the invention as set forth in the appended claims.

### Reference Signs List

1 Heat-generating layer
11 Oxidizable metal
12 Carbon component
2 Water-retention layer
21 Water-absorbent polymer
22 Thermoplastic resin
22S Dispersion layer
23 Thermoplastic water-absorbent resin
23S Layer of thermoplastic water-absorbent resin
24 Water-soluble resin
3 First sheet
4 Second sheet
5 Closed space
6 Flap portion
10 Warming device

## Claims

1. A warming device, comprising a first sheet, a second sheet, a heat-generating layer, and a water-retention layer,
the heat-generating layer comprising an oxidizable metal, a carbon component, and water, and
the water-retention layer comprising one, or two or more selected from: a dispersion layer that is a layer of a thermoplastic resin in which a water-absorbent polymer is dispersed; and a layer of a thermoplastic water-absorbent resin,
wherein the water-retention layer retains water,
the heat-generating layer and the water-retention layer are adjacent to each other and are disposed in a closed space, the closed space being sandwiched between the first sheet and the second sheet,
the first sheet is air-permeable, and
in a plan view, the heat-generating layer and the water-retention layer are smaller in area than the first sheet and the second sheet, and a flap portion where the first sheet and the second sheet overlap is provided outside outer peripheral edges of the heat-generating layer and the water-retention layer.

2. The warming device according to claim 1, wherein the heat-generating layer is in contact with the first sheet.

3. The warming device according to claim 1, wherein the water-retention layer is in contact with the first sheet, and in a plan view from a side of the first sheet, an area of the water-retention layer is smaller than an area of the heat-generating layer.

4. The warming device according to any one of claims 1 to 3, wherein a plan view area of a fiber material in the water-retention layer is, relative to a plan view area of an entire water-retention layer, 0% or more and 10% or less, preferably 5% or less, and more preferably 3% or less.

5. The warming device according to any one of claims 1 to 4, wherein a thickness of the water-retention layer is 140 µm or more and 500 µm or less, preferably 150 µm or more and 400 µm or less, and more preferably 200 µm or more and 300 µm or less.

6. The warming device according to any one of claims 1 to 5, wherein the water-retention layer comprises the dispersion layer, and
a content proportion of the thermoplastic resin relative to a mass of the entire water-retention layer is 20 mass% or more and 60 mass% or less, preferably 30 mass% or more, and more preferably 40 mass% or more.

7. The warming device according to any one of claims 1 to 6, wherein the water-retention layer comprises the dispersion layer, and
for sections each comprising a thickness cross-section cut out with a width of 1 mm in a planar direction on the water-retention layer, a standard deviation of area values of the water-absorbent polymer present in thickness cut surfaces of five of the sections is 0 or more and 10 or less, preferably 5 or less, and more preferably 3 or less.

8. The warming device according to any one of claims 1 to 7, wherein the water-retention layer comprises the dispersion layer, and the dispersion layer comprises one, or two or more selected from a water-soluble resin and sodium chloride.

9. The warming device according to claim 8, wherein a total content proportion of the water-soluble resin and the sodium chloride relative to the mass of the entire water-retention layer is 2 mass% or more and 30 mass% or less, and preferably 25 mass% or less.

10. The warming device according to any one of claims 1 to 9, wherein the water-retention layer comprises the dispersion layer, and the dispersion layer comprises a surfactant.

11. The warming device according to claim 10, wherein a content proportion of the surfactant relative to the mass of the entire water-retention layer is 0.1 mass% or more and 3 mass% or less, and preferably 1 mass% or less.

12. The warming device according to any one of claims 1 to 11, wherein the water-retention layer comprises the dispersion layer, and the dispersion layer comprises an arrangement configuration in which the water-absorbent polymers adjacent to each other are in contact with each other.

13. The warming device according to claim 12, wherein the water-absorbent polymers comprise polymers exposed to a surface of the dispersion layer, the surface on a side of the heat-generating layer.

14. The warming device according to any one of claims 1 to 13, wherein the water-retention layer comprises the dispersion layer, and the dispersion layer comprises a cracked portion at a surface on a side of the heat-generating layer.

15. The warming device according to any one of claims 1 to 14, wherein a thickness of the warming device is 0.5 mm or more and 1.2 mm or less, preferably 1.0 mm or less, and more preferably 0.8 mm or less.

16. The warming device according to any one of claims 1 to 15, wherein a maximum temperature of the warming device during heat generation is 45°C or higher and 75°C or lower, preferably 60°C or higher and 73°C or lower, and more preferably 65°C or higher and 70°C or lower.

17. The warming device according to any one of claims 1 to 16, wherein time required for the warming device to reach 45°C from 35°C is more than 0 minutes and 5 minutes or less, preferably 3 minutes or less, and more preferably 1 minute or less.

18. A method for manufacturing a warming device, the method comprising:
applying a coating material comprising an oxidizable metal, a carbon component, and water onto one surface of a first raw material sheet;
applying: a slurry of a molten thermoplastic resin comprising a water-absorbent polymer; and/or a molten thermoplastic water-absorbent resin onto one surface of a second raw material sheet; and
bonding the first raw material sheet and the second raw material sheet in such a way that coated surfaces of the first raw material sheet and the second raw material sheet face each other,
wherein one or both selected from the first raw material sheet and the second raw material sheet are air-permeable sheets.

19. The method for manufacturing a warming device according to claim 18, wherein
the coating material and either the slurry or the molten thermoplastic water-absorbent resin, or both the slurry and the molten thermoplastic water-absorbent resin, are intermittently applied in a machine direction; and
the first raw material sheet and the second raw material sheet outside coated portions in the machine direction are sealed.

20. The method for manufacturing a warming device according to claim 18 or 19, wherein
coating widths, in a cross direction perpendicular to the machine direction, of the coating material and either the slurry or the molten thermoplastic water-absorbent resin, or both the slurry and the molten thermoplastic water-absorbent resin, are made narrower than widths of the first raw material sheet and the second raw material sheet, and
the first raw material sheet and the second raw material sheet outside the coated portions in the cross direction are sealed.

21. The method for manufacturing a warming device according to any one of claims 18 to 20, wherein the coating material and either the slurry or the molten thermoplastic water-absorbent resin, or both the slurry and the molten thermoplastic water-absorbent resin, are intermittently applied in a cross direction perpendicular to the machine direction.

22. The method for manufacturing a warming device according to any one of claims 18 to 21, wherein the method comprises applying the slurry onto one surface of the second raw material sheet, and
an average particle diameter of the water-absorbent polymer is 1 µm or more and 360 µm or less, preferably 10 µm or more and 100 µm or less, and more preferably 40 µm or more and 80 µm or less.

23. The method for manufacturing a warming device according to any one of claims 18 to 22, wherein
a content proportion of sodium chloride in the coating material is 0 mass% or more and 30 mass% or less, preferably 17 mass% or less, and more preferably 10 mass% or less.

24. The method for manufacturing a warming device according to any one of claims 18 to 23, wherein the method comprises applying the slurry onto one surface of the second raw material sheet, and
the slurry comprises one, or two or more selected from a water-soluble resin and sodium chloride.

25. The method for manufacturing a warming device according to any one of claims 18 to 24, wherein the method comprises:
applying the slurry onto one surface of the second raw material sheet; and
pressing the applied slurry with a roll.

26. The method for manufacturing a warming device according to any one of claims 18 to 25, wherein the method comprises:
applying the slurry onto one surface of the second raw material sheet; and
a ratio of a mass proportion of the thermoplastic resin to a mass proportion of the water-absorbent polymer, the sodium chloride, and the water-soluble resin, (mass proportion of the thermoplastic resin/(mass proportion of the water-absorbent polymer + mass proportion of the sodium chloride + mass proportion of the water-soluble resin)), accounting for a total mass of the slurry, is 0.5 or more and 5 or less, preferably 0.8 or more and 3 or less, and more preferably 1.3 or more and 2 or less.
